# DEMANDE DE BREVET EUROPEEN

(11) **EP 4 039 354 A1**
(43) Date de publication de la demande: **10.08.2022**
(21) Numéro de dépôt: 22155197.1
(22) Date de dépôt: 04.02.2022
(51) Int. Cl.: B01D 53/04, B01D 53/00

(54) **PURIFICATION DE FLUX GAZEUX PAR ADSORPTION AVEC PRÉ-RÉGÉNÉRATION EN BOUCLE FERMÉE**

(30) Priorité: 05.02.2021 FR 2101099
(71) Demandeur: L'Air Liquide, société anonyme pour l'Étude et l'Exploitation des procédés Georges Claude, 75007 Paris (FR)
(72) Inventeur: Zick, Golo, 38360 Sassenage (FR)
(74) Mandataire: Air Liquide

(57) **Abrégé**

Procédé de production de biométhane liquide mettant en œuvre une installation de production de biométhane liquide comprenant :
- Un digesteur permettant de produire du biogaz,
- Une unité de séparation membranaire permettant de produire du biométhane à partir du biogaz,
- Une installation P de purification du biométhane par adsorption de type TSA permettant de produire du biométhane purifié à partir du biométhane sortant de l'unité de séparation membranaire, et
- Une unité de liquéfaction du biométhane purifié ;
le procédé comprenant les étapes suivantes :
a) Digestion anaérobique de biomasse de manière à produire du biogaz,
b) Séparation membranaire du biogaz de manière à produire du biométhane,
c) Purification du biométhane dans l'installation P de purification, et
d) Liquéfaction du biométhane purifié.

## Description

La présente invention est relative à un procédé de production de biométhane liquide mettant en œuvre ou utilisant une installation de production de biométhane liquide comprenant une installation de purification d'un flux gazeux par adsorption de type TSA. L'invention peut concerner également un procédé de purification d'un flux gazeux par adsorption de type TSA comprenant une étape de pré-régénération ne mettant en œuvre aucun gaz de régénération extérieur à l'adsorbeur en pré-régénération et à l'installation mise en œuvre par le procédé.

Dans beaucoup d'applications industrielles la nécessité d'une séparation en espèces d'un flux gazeux se pose. Pour cela différentes méthodologies ont été développées, qui se différencient par leur domaine optimal d'opération, par exemple les systèmes membranaires sont en général bien adaptés pour faire une séparation primaire à moindre coût sans atteindre des puretés importantes. Si ces dernières sont nécessaires, par exemple pour une liquéfaction en aval, les systèmes d'adsorption sont couramment utilisés. Ces systèmes se chargent en impuretés et nécessitent donc une régénération cyclique, qui peut se faire par variation de pression ou de température ou encore d'une combinaison des deux effets. Dans le cas d'une impureté avec un taux faible, on préfère en général utiliser une régénération qui se base principalement sur une variation de température (TSA = Temperature Swing Adsorption = adsorption avec variation de température), sans pour autant être insensible aux variations de pressions, et qui s'opère donc sur un temps de cycle relativement long dû à l'inertie thermique du système dans sa globalité. Afin d'assurer une opération en continu, on opère souvent avec plusieurs bouteilles d'adsorption en parallèle qui se trouvent à différentes étapes d'adsorption ou de régénération.

Classiquement, un cycle de procédé TSA comporte les étapes suivantes:
a) purification du flux gazeux par adsorption des impuretés à une pression supérieure à la pression atmosphérique et proche de la température ambiante (aussi appelé phase de production),
b) dépressurisation de l'adsorbeur jusqu'à la pression atmosphérique,
c) régénération de l'adsorbant à pression atmosphérique au moyen d'un gaz de régénération réchauffé jusqu'à une température habituellement entre 100 et 300°C au moyen d'un ou plusieurs échangeurs thermiques,
d) refroidissement à température ambiante de l'adsorbant, et
e) repressurisation de l'adsorbeur avec du flux gazeux purifié issu, par exemple, d'un autre adsorbeur se trouvant en phase de production.

Pour la régénération, afin de réaliser la variation de température, c.-à-d. l'apport d'énergie pour chauffer, on utilise habituellement un flux de gaz chaud comme vecteur, injecté dans la bouteille en contre-sens du flux d'adsorption. On choisit alors pour ce gaz de régénération en général un gaz inerte qui ne s'adsorbe très peu afin de minimiser son impact sur la capacité d'adsorption et de ne pas polluer les parties qui sont resté sans contact avec l'impureté lors de la phase de production. Dans certains procédés on dispose d'un gaz d'évent utilisable, par exemple de l'azote qui a préalablement servi comme source froide sous forme liquide ou une partie de la production qui doit être évacuée car chargée d'une autre impureté non pertinente pour l'adsorption en question. Dans d'autres procédés, qui sont justement le sujet de la présente invention, on ne dispose pas d'une telle source de gaz. Dans ce cas il faut soit mettre à disposition une source de gaz dédié pour la régénération, par exemple un stockage d'azote liquide avec un évaporateur atmosphérique, soit utiliser à cette fin une fraction du flux épuré. Cette dernière peut parfois être recyclée plus en amont dans le procédé, mais dans tous les cas il y a des surcoûts à escompter pour les pertes et/ou le surdimensionnement et l'opération des unités en amont.

Ils existent des systèmes de réchauffage direct qui transmettent la chaleur par conduction à travers le lit, mais ces systèmes ont l'inconvénient d'être assez limités en puissance transférable et demandent donc un temps de régénération relativement long. En plus les impuretés désorbées doivent être évacuées de l'adsorbant ce qui nécessite de nouveau un gaz vecteur ou un système de pompage sur la bouteille.

Partant de là, un problème qui se pose est de proposer une nouvelle façon de régénérer un adsorbant quand aucun gaz de régénération n'est facilement disponible. Les pertes et recyclages sont à minimiser autant que possible.

Une solution de la présente invention est une installation P de purification d'un flux gazeux, par exemple du biométhane, du gaz naturel ou du gaz de fumée, par adsorption de type TSA comprenant :
- Au moins deux adsorbeurs (A, B ou C) suivant, en décalage, un cycle où se succède une phase d'adsorption et une phase de régénération, la phase de régénération comportant une étape de dépressurisation, une étape de pré-régénération et une étape de régénération,
- Un moyen de dépressurisation 5 permettant de dépressuriser l'adsorbeur au début de la phase de régénération,
- Un circulateur de gaz 2 permettant de faire circuler le gaz compris ou contenu dans l'adsorbeur en pré-régénération dans une boucle fermée, et
- Un réchauffeur 3 permettant de chauffer le gaz circulant dans la boucle fermée.

L'installation P de purification peut également comprendre un moyen permettant d'isoler l'adsorbeur en pré-régénération du reste de l'installation P.

Autrement dit la boucle fermée peut comprendre, par exemple, dans le sens de circulation du gaz, l'adsorbeur en pré-régénération, le circulateur de gaz, le réchauffeur, et la tuyauterie permettant de connecter les différents éléments et de faire circuler le gaz entre ces différents éléments.

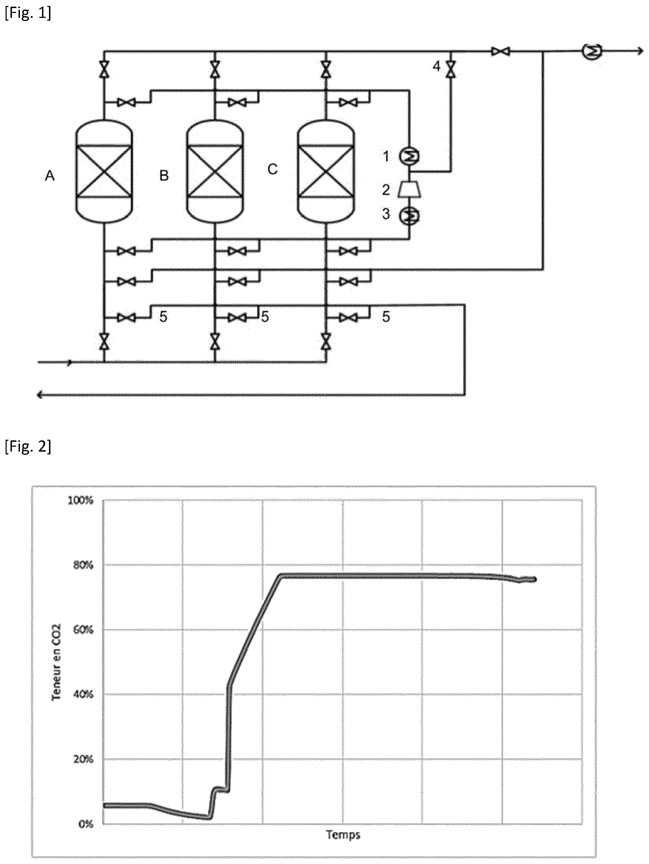

Un exemple d'installation P de purification selon l'invention est représenté figure 1.

Par « isoler l'adsorbeur » on entend la coupure d'une possibilité d'échange fluidique entre la boucle fermée de pré-régénération et le reste du cycle, typiquement par des vannes. Il est néanmoins possible de mettre en relation la boucle fermée de pré-régénération avec le reste du cycle à des endroits choisis afin de réguler temporairement la pression de la boucle par chargement ou déchargement de gaz.

Par « dépressurisation » on entend ventiler ou recycler de la haute pression à la basse pression. L'étape de dépressurisation s'effectue de préférence avant l'étape de pré-régénération.

Par « pré-régénération » on entend par exemple le processus suivant : le flux gazeux qui se trouve dans l'adsorbeur est mis en circulation dans un circuit fermé ou une boucle fermée chauffé(e) afin d'augmenter la température de l'adsorbant et de l'adsorbeur jusqu'à la température cible de régénération ou une température proche de celle-ci. Pendant la pré-régénération, la désorption de l'impureté, par exemple du CO2, qui avait été captée dans l'adsorbeur, a lieu en raison d'une capacité d'adsorption plus faible à une pression plus basse et une température plus élevée, mais la désorption n'est pas suffisante pour le niveau de régénération nécessaire. La désorption est limitée car la pression partielle du CO₂ augmente en conséquence. La masse circulante augmente du fait du CO₂ désorbé. Par conséquent, de temps en temps, une ventilation peut être nécessaire pour limiter l'augmentation de la pression. De préférence, pendant la pré-régénération la pression est maintenue constante en déchargeant la boucle si nécessaire.

Par « régénération » on entend une circulation d'un fluide, en particulier un gaz de régénération, pour désorber et/ou pousser l'impureté, par exemple le CO₂, désorbé de l'adsorbant et de l'adsorbeur. Le fluide ou le gaz de régénération est chauffé dans un premier temps pour tenir compte de la chaleur de désorption, et refroidi, par exemple à température ambiante de l'adsorbant. Le fluide ou le gaz de régénération peut comprendre une faible quantité de l'impureté, par exemple du CO₂, qui a été adsorbée dans l'adsorbeur lors de la phase d'adsorption. Par exemple, le fluide ou le gaz de régénération peut comprendre une quantité de l'impureté, par exemple du CO₂, inférieur ou égale à la spécification de taux d'impureté dans le produit final ou le flux gazeux purifié (le produit final est par exemple le biométhane purifié, le gaz naturel purifié ou le gaz de fumée purifié). De préférence, le fluide ou le gaz de régénération ne contient pas de CO₂.

Par « gaz compris ou contenu dans l'adsorbeur » on entend le gaz compris ou contenu dans l'adsorbeur à la fin de la phase d'adsorption et après l'étape de dépressurisation de la phase de régénération. Le gaz compris ou contenu dans l'adsorbeur est par exemple le gaz qui s'y trouve au moment du lancement de la pré-régénération, c'est-à-dire, le gaz à purifier avec l'adsorbat, qui a été adsorbé dans l'adsorbeur, typiquement du CO₂, en particulier lorsque le flux gazeux à purifier est du biométhane.

Par « circulateur de gaz » on entend un ventilateur ou un compresseur avec par exemple un faible taux de compression (typiquement inférieur à 1,5), juste suffisant pour vaincre les pertes de charge du lit et pour maintenir un écoulement en boucle fermée.

Par « boucle fermée » de pré-régénération on entend un circuit fermé où aucune source de gaz externe n'est utilisée. Il peut rester une pression résiduelle après l'étape de dépressurisation à un certain niveau de pression.

Ces équipements servent à réaliser une régénération préliminaire ou pré-régénération en boucle fermée. En effet, souvent plus que la moitié de l'énergie apportée lors d'une régénération ne sert qu'à réchauffer la matière de l'adsorbant même et le matériel de la bouteille ou de l'adsorbeur, sans que cela soit directement utile dans l'objectif final de désorber le gaz piégé sur l'adsorbant. Ce n'est qu'une inertie thermique à vaincre et qui ne demande pas une qualité de gaz spécifique.

En balayant l'adsorbeur en boucle fermée, on peut finement réguler l'apport de chaleur qui est fonction du débit et de la température. En même temps, le gaz circulant dans la boucle fermée se concentre en impureté au cours du temps car la capacité d'adsorption baisse avec la montée de température.

Cette impureté qui a été arrêtée sur l'adsorbant pendant la phase d'adsorption et qui est désorbée pendant la pré-régénération et la régénération peut être par exemple choisie parmi le CO2, un hydrocarbure, de l'eau ou un gaz de l'air.

Selon le cas, l'installation P de purification selon l'invention peut présenter une ou plusieurs des caractéristiques ci-dessous :
- Le moyen de dépressurisation 5 comprend une vanne de régulation de pression. La vanne de régulation de pression permet de lier la boucle fermée vers l'évent ou le recycle afin de pouvoir maintenir la pression qui varie en fonction du réchauffement et de la désorption de l'impureté, par exemple du CO₂, qui avait été captée dans l'adsorbeur,
- L'installation P de purification comprend un refroidisseur 1 permettant de refroidir le gaz circulant dans la boucle fermée en amont du circulateur de gaz. Ce refroidisseur est utilisé par exemple lorsque la température du gaz circulant dans la boucle fermée est trop élevée pour le fonctionnement du circulateur de gaz. La présence du refroidisseur ou le niveau du refroidissement dépendent de la technologie et du design du circulateur de gaz. Typiquement, le circulateur de gaz permettra de refroidir le gaz circulant dans la boucle fermée à une température inférieure à 80°C, de préférence inférieure à 60°C. Dans ce cas, la boucle fermée comprendra dans le sens de circulation du gaz, l'adsorbeur en pré-régénération, le circulateur de gaz, le réchauffeur, et la tuyauterie permettant de connecter les différents éléments et de faire circuler le gaz entre ces différents éléments.
- le flux gazeux comprend au moins 90% de CH₄, de préférence au moins 97% de CH₄ et les adsorbeurs comprennent un matériau actif qui va adsorber au moins une partie du CO₂.
- le flux gazeux peut comprendre au moins 60% de N₂ lorsque le flux gazeux est un gaz de fumée.
- L'installation P de purification comprend un moyen d'introduction 4 d'un gaz de régénération dans l'adsorbeur suivant ou après l'étape de pre-régénération. Notons que le gaz de régénération correspondra de préférence à une partie du flux gazeux purifié. Aussi le moyen d'introduction sera de préférence un moyen permettant d'introduire une partie du flux gazeux purifié dans l'adsorbeur.
- L'installation P de purification comprend un moyen de chauffage 3 du gaz de régénération. Notons que le moyen de chauffage est de préférence le même réchauffeur que celui permettant de chauffer le gaz circulant dans la boucle fermée.
- L'installation P de purification comprend au moins trois adsorbeurs A, B et C.
- L'installation P de purification comprend un moyen permettant de refroidir l'adsorbeur avant l'adsorption. Ce moyen sera de préférence un moyen permettant d'envoyer la totalité ou une grande partie du gaz purifié par un autre adsorbeur dans l'adsorbeur venant d'être régénéré.

A titre d'exemple, l'installation P de purification selon l'invention pourra être insérée dans une installation de production de biométhane liquide comprenant :
- Un digesteur permettant de produire du biogaz,
- Une unité de séparation membranaire permettant de produire du biométhane à partir du biogaz,
- L'installation P de purification selon l'invention permettant de produire du biométhane purifié à partir du biométhane sortant de l'unité de séparation membranaire, et
- Une unité de liquéfaction du biométhane purifié.

La présente invention a également pour objet un procédé de purification d'un flux gazeux, par exemple du biométhane, du gaz naturel ou du gaz de fumée, par adsorption de type TSA, mettant en œuvre une installation P de purification selon l'invention et comprenant avant la pré-régénération, une étape de dépressurisation d'un adsorbeur, et pendant la pré-régénération une étape d'isolation de l'adsorbeur dépressurisé dans une boucle fermée et une étape de chauffage et de circulation du gaz se trouvant dans l'adsorbeur isolé dans la boucle fermée. Ledit gaz se trouvant dans l'adsorbeur est par exemple le gaz qui s'y trouve au moment du lancement de la pré-régénération, c'est-à-dire, le gaz à purifier avec l'adsorbat, qui a été adsorbé dans l'adsorbeur, typiquement du CO₂, en particulier lorsque le flux gazeux à purifier est du biométhane.

Selon le cas, le procédé de purification selon l'invention peut présenter une ou plusieurs des caractéristiques ci-dessous :
- La pré-régénération comprend une étape de refroidissement du gaz circulant dans la boucle fermée en amont du circulateur de gaz.
- à l'étape de chauffage le gaz se trouvant dans l'adsorbeur est chauffé à une température comprise entre 100°C et 300°C.
- à l'étape de dépressurisation le gaz se trouvant dans l'adsorbeur isolé est dépressurisé à une pression comprise entre 1 et 3 bar, de préférence entre 1,5 et 2,5 bar ; la dépressurisation ou ventilation peut être réactivée temporairement lors de l'étape de pré-régénération afin de stabiliser la pression d'opération.
- le procédé comprend après la pré-régénération l'introduction d'un gaz de régénération dans l'adsorbeur. Le gaz de régénération permettre de désorber et/ou pousser l'impureté, par exemple le CO₂, désorbé de l'adsorbant et de l'adsorbeur. Le gaz de régénération est chauffé dans un premier temps pour tenir compte de la chaleur de désorption, et refroidi, par exemple à température ambiante de l'adsorbant. Le gaz de régénération peut comprendre une faible quantité de l'impureté, par exemple du CO₂, qui a été adsorbée dans l'adsorbeur lors de la phase d'adsorption. Par « faible quantité » on entend inférieur ou égale à la spécification de taux d'impureté dans le produit final ou le flux gazeux purifié, ce produit final peut être par exemple le biométhane purifié. La spécification de taux d'impureté maximal dans le produit final est par exemple de 1ppm à 1%. Par exemple, la spécification de taux du CO₂ maximal dans le biométhane purifié est par exemple de l'ordre de 50ppm. De préférence, le gaz de régénération ne contient pas de CO₂.
- le gaz de régénération est une partie du flux gazeux purifié par l'installation P de purification selon le procédé de purification. De préférence, cette partie représentera moins de 10%, de préférence encore moins de 5% du débit du flux gazeux purifié.
- le gaz de régénération est chauffé à une température comprise entre 100°C et 300°C.
- la pré-régénération représente entre 5% et 15% du cycle suivi par chaque adsorbeur, c'est-à-dire la fraction de la durée d'une étape de pré-régénération représente entre 5% et 15% par rapport à la durée total d'une cycle de purification ou de régénération complet.

A titre d'exemple, le procédé de purification selon l'invention pourra être compris dans un procédé de production de biométhane liquide mettant en œuvre l'installation de production de biométane liquide décrite précédemment et comprenant les étapes suivantes :
a) Digestion anaérobique de biomasse de manière à produire du biogaz,
b) Séparation membranaire du biogaz de manière à produire du biométhane,
c) Purification du biométhane dans l'installation P de purification selon le procédé de purification du biométhane tel que défini précédemment, et
d) Liquéfaction du biométhane purifié.

L'étape a) est réalisée de préférence par le digesteur. L'étape b) de séparation est réalisée de préférence par l'unité de séparation membranaire.

L'invention va à présent être décrite plus en détail à l'aide de l'exemple ci-dessous.

Nous prenons - sans limitation à ce procédé ou cette application spécifique - comme exemple la purification d'un flux de Méthane venant d'une pré-purification membranaire avec une impureté de 2% de Dioxyde de Carbone. Le méthane est destiné à être liquéfié, donc la limite admissible en dioxyde de carbone est de l'ordre de 50ppm afin d'en éviter la solidification. Dans ce procédé aucune source d'un gaz de régénération n'est naturellement disponible, puisque la liquéfaction se fait par un cycle frigorifique fermé et le méthane est liquéfié entièrement.

La succession des différentes étapes de l'opération est classique et n'est pas répété ici sauf pour la régénération qui est innovante.

Après la dépressurisation vers un niveau de pression qui permet une phase de régénération, en particulier une étape de pré-régénération, sur un niveau de pression optimale sous respect du risque d'attrition, de taille du circulateur et la vitesse de l'apport de chaleur (1 à 3 bar), le circuit comprenant l'adsorbeur dépressurisé et chargé en CO₂ ainsi que le circulateur et son réchauffeur sont isolé du reste du système. Une vanne de régulation de pression lie néanmoins ce circuit vers l'évent ou le recycle afin de pouvoir maintenir la pression qui varie en fonction du réchauffement et de la désorption du CO₂. La pré-régénération en boucle fermée est de préférence réalisée à la pression la plus élevée possible, éventuellement limitée par la vanne de régulation de pression, afin d'augmenter la capacité calorifique disponible pour le réchauffement et de limiter le risque d'attrition pour raccourcir cette étape de pré-régénération.

En balayant l'adsorbeur en boucle fermée, on peut finement réguler l'apport de chaleur qui est fonction du débit et de la température. En même temps le gaz se concentre en CO₂ au cours du temps comme on peut le voir sur la figure 2 tandis que l'adsorbant contenu dans l'adsorbeur est déchargé en CO₂.

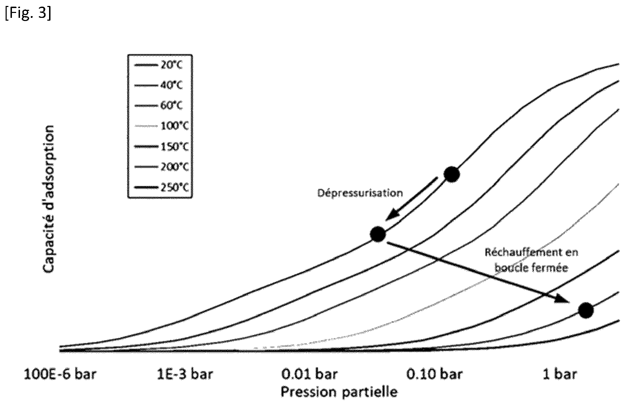

Contre toute attente, cette haute teneur en CO2 ne va pas charger à nouveau l'adsorbant contenu dans l'adsorbeur, car comme la pression totale a été affaiblie la pression partielle de CO2 ne monte pas beaucoup. Par contre la montée en température baisse la capacité d'adsorption de sorte que le chargement de l'adsorbant a déjà fortement diminué quand la température finale qui est visée avec la boucle est atteinte. Ce phénomène est représenté figure 3.

Aussi, la pré-régénération ne sert pas uniquement à réchauffer les parties inertes de l'adsorbeur, mais commence déjà la désorption du CO2 de l'adsorbant d'une manière importante.

Une fois la température visée atteinte, en générale comprise entre 100°C et 300°C, les étapes de régénération finale peuvent commencer. Pour cela un gaz, plus précisément un gaz de régénération, comprenant le moins de CO₂ possible et qui ne s'adsorbe que très faiblement est nécessaire. De préférence, le gaz de régénération ne contient pas de CO₂. L'étape de régénération finale est réalisée à la pression la plus basse possible pour bénéficier au maximum de la faible pression partielle de l'impureté, par exemple du CO₂.

On choisit donc un prélèvement dans le flux gazeux purifié, en particulier le biométhane purifié, qui est envoyé à la liquéfaction. Il s'avère que le prélèvement nécessaire représente moins de 10% voire moins de 5% du débit du flux gazeux purifié, ce qui est remarquablement faible comparé à l'état de l'art. On ne peut pas envoyer directement ce gaz prélevé dans l'adsorbeur chaud, car la baisse de température de l'adsorbant mènerait à une capacité d'adsorption plus élevée et donc à la ré-adsorption du CO₂ qui s'y trouverait encore. Il faut donc dans un premier temps réchauffer ce gaz pour créer une zone bien régénérée et sans impuretés résiduelles dans la phase gazeuse. Une fois cette zone établie, on arrête le réchauffeur et on pousse par effet piston cette zone chaude et propre à travers tout l'adsorbeur. On atteint donc un état dans lequel l'intégralité de l'adsorbeur est bien régénéré mais encore très chaud (température proche de la température de chauffe maximale visée). Afin d'éviter une perte de CH₄, ce débit de régénération peut être renvoyé pour recyclage vers le système membranaire en amont.

Avant de recommencer une phase d'adsorption, l'adsorbeur et l'adsorbant doivent être refroidi. Pour cela, on peut envoyer la totalité ou une grande partie du gaz purifié par un autre adsorbeur qui est envoyé à travers l'adsorbeur chaud mais propre. Cette manière de procéder est bien moins coûteuse et plus facile à mettre en œuvre qu'un système avec un flux spécifique pour le refroidissement de l'adsorbeur.

La solution selon l'invention permet de générer des débits et des compositions de production qui sont suffisamment stables sur toute la durée d'un cycle. Elle n'a donc que très peu d'impact sur les unités en amont et en aval.

## Revendications

1. Procédé de production de biométhane liquide mettant en œuvre une installation de production de biométhane liquide comprenant :
- Un digesteur permettant de produire du biogaz,
- Une unité de séparation membranaire permettant de produire du biométhane à partir du biogaz,
- Une installation P de purification du biométhane par adsorption de type TSA permettant de produire du biométhane purifié à partir du biométhane sortant de l'unité de séparation membranaire, et
- Une unité de liquéfaction du biométhane purifié ;
le procédé comprenant les étapes suivantes :
a) Digestion anaérobique de biomasse de manière à produire du biogaz,
b) Séparation membranaire du biogaz de manière à produire du biométhane,
c) Purification du biométhane dans l'installation P de purification,
ladite installation P de purification comprennent :
- Au moins deux adsorbeurs (A, B ou C) suivant, en décalage, un cycle où se succède une phase d'adsorption et une phase de régénération, la phase de régénération comportant une étape de dépressurisation, une étape de pré-régénération et une étape de régénération,
- Un moyen de dépressurisation (5) permettant de dépressuriser l'adsorbeur au début de la phase de régénération,
- Un circulateur de gaz (2) permettant de faire circuler le gaz compris dans l'adsorbeur en pré-régénération dans une boucle fermée, et
- Un réchauffeur (3) permettant de chauffer ledit gaz circulant dans la boucle fermée ; la purification du biométhane comprenant, avant la pré-régénération, une étape de dépressurisation d'un adsorbeur, et, pendant la pré-régénération, une étape d'isolation de l'adsorbeur dépressurisé dans une boucle fermée et une étape de chauffage et de circulation du gaz se trouvant dans l'adsorbeur isolé dans la boucle fermée ; et
d) Liquéfaction du biométhane purifié.

2. Procédé selon la revendication 1, **caractérisée en ce que** le moyen de dépressurisation (5) compris dans l'installation P de purification comprend une vanne de régulation de pression.

3. Procédé selon l'une des revendications 1 ou 2, **caractérisée en ce que** l'installation P de purification comprend un refroidisseur (1) permettant de refroidir le gaz circulant dans la boucle fermée en amont du circulateur de gaz.

4. Procédé selon l'une quelconque des revendications 1 à 3, **caractérisée en ce que** le biométhane comprend au moins 90% de CH₄, de préférence au moins 97% de CH₄ et les adsorbeurs comprennent un matériau actif qui va adsorber au moins une partie du CO₂.

5. Procédé selon l'une quelconque des revendications 1 à 4, **caractérisée en ce que** l'installation P de purification comprend un moyen d'introduction (4) d'un gaz de régénération dans l'adsorbeur après l'étape de pré-régénération.

6. Procédé selon la revendication 5, **caractérisée en ce que** l'installation P de purification comprend un moyen de chauffage (3) du gaz de régénération.

7. Procédé selon l'une quelconque des revendications 1 à 6, **caractérisé en ce que** la pré-régénération comprend une étape de refroidissement du gaz circulant dans la boucle fermée en amont du circulateur de gaz.

8. Procédé selon l'une quelconque des revendications 1 à 7, **caractérisé en ce qu'**à l'étape de chauffage et de circulation de la purification c) du biométhane, le gaz se trouvant dans l'adsorbeur est chauffé à une température comprise entre 100°C et 300°C.

9. Procédé selon l'une quelconque des revendications 1 à 8, **caractérisé en ce qu'**à l'étape de dépressurisation de la purification c) du biométhane, le gaz se trouvant dans l'adsorbeur isolé est dépressurisé à une pression comprise entre 1 et 3 bar, de préférence entre 1,5 et 2,5 bar.

10. Procédé selon l'une quelconque des revendications 1 à 9, **caractérisé en ce que** la purification c) du biométhane comprend après la pré-régénération l'introduction d'un gaz de régénération dans l'adsorbeur.

11. Procédé selon la revendication 10, **caractérisé en ce que** le gaz de régénération est une partie du biométhane purifié par l'installation P de purification.

12. Procédé selon l'une des revendications 10 ou 11, **caractérisé en ce que** le gaz de régénération est chauffé à une température comprise entre 100°C et 300°C.

13. Procédé selon l'une quelconque des revendications 1 à 12, **caractérisé en ce que** la pré-régénération représente entre 5% et 15% du cycle suivi par chaque adsorbeur.
